(19) **Europäisches Patentamt / European Patent Office / Office européen des brevets**

(11) **EP 1 480 695 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(21) Anmeldenummer: **03706352.6**

(22) Anmeldetag: **09.01.2003**

(51) Int Cl.:
***A61M 1/36*** **(2006.01)**

(86) Internationale Anmeldenummer:
**PCT/EP2003/000126**

(87) Internationale Veröffentlichungsnummer:
**WO 2003/074109 (12.09.2003 Gazette 2003/37)**

(54) **VORRICHTUNG ZUR BESTIMMUNG DES HÄMATOKRIT UND/ODER BLUTVOLUMENS**

DEVICE FOR DETERMINING THE HEMATOCRIT AND/OR BLOOD VOLUME

DISPOSITIF DE DETERMINATION DU TAUX D'HEMATOCRITE ET/OU DU VOLUME SANGUIN

(84) Benannte Vertragsstaaten:
**AT BE BG CH CY CZ DE DK EE ES FI FR GB GR HU IE IT LI LU MC NL PT SE SI SK TR**

(30) Priorität: **07.03.2002 DE 10210009**

(43) Veröffentlichungstag der Anmeldung:
**01.12.2004 Patentblatt 2004/49**

(73) Patentinhaber: **Fresenius Medical Care Deutschland GmbH**
**61352 Bad Homburg v.d.H. (DE)**

(72) Erfinder:
- **ZHANG, Wei**
  **97421 Schweinfurt (DE)**
- **BRAUER, Helge**
  **97469 Gochsheim (DE)**
- **SPICKERMANN, Reiner**
  **97535 Burghausen (DE)**
- **MÜLLER, Carsten**
  **97502 Euerbach (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**DE-A- 4 024 434**      **US-A- 6 061 590**
**US-B1- 6 217 539**

**Beschreibung**

Die Erfindung betrifft eine Vorrichtung zur extrakorporalen Blutbehandlung mit einem extrakorporalen Blutkreislauf und einer Einrichtung zur Bestimmung des Hämatokrit und/oder Blutvolumens.

Zur Entfernung von hampflichtigen Substanzen und zum Flüssigkeitsentzug werden beim akuten und chronischen Nierenversagen verschiedene Verfahren zur apparativen Blutreinigung bzw. Blutbehandlung eingesetzt. Bei der Hämodialyse (HD) überwiegt der diffusive Stofftransport, während bei der Hämofiltration (HF) ein konvektiver Stofftransport über die Membran stattfindet. Eine Kombination aus beiden Verfahren ist die Hämodiafiltration (HDF).

Ein zu hoher bzw. zu schneller Flüssigkeitsentzug während der Hämodialyse kann eine ggf. rapide Blutvolumenabnahme hervorrufen, welche häufig zum akuten Blutdruckabfall (Hypotonie) beim Patienten führt.

Die Hypotonie stellt eine der Hauptkomplikationen bei der Blutbehandlung dar. Es gibt verschiedene Lösungen für dieses Problem. Zum einen sind Blutdruckmonitore bekannt, die eine Änderung des Blutdrucks kontinuierlich überwachen und die Ultrafiltration in Abhängigkeit von der Blutdruckänderung regeln. Zum anderen sind Blutvolumenmonitore bekannt, die das relative Blutvolumen während der Dialysebehandlung messen und eine Regelung der Ultrafiltration in Abhängigkeit vom relativen Blutvolumen vornehmen.

Die DE-C-197 46 377 beschreibt eine Vorrichtung zur Messung des Blutdrucks, die auf der Erfassung der Ausbreitungsgeschwindigkeit der sich über das arterielle Gefäßsystem des Patienten fortpflanzenden Pulswellen beruht, die durch dessen Herzkontraktionen erzeugt werden. Die Vorrichtung erlaubt zwar eine kontinuierliche, nicht invasive Messung des Blutdrucks, nachteilig ist jedoch, dass die Pulswellenlaufzeit vom Hämatokrit (HKT) abhängig ist.

Die DE-A-40 24 434 wird als nächst kommender Stand der Technik augesehen. Sie beschreibt eine Vorrichtung zur Ultrafiltrationsregelung, bei der zum Bestimmen des relativen Blutvolumens der Druck im extrakorporalen Kreislauf gemessen wird. Die gemessenen Druckwerte werden in zeitlicher Abfolge gespeichert und aus der Veränderung des Druckwertes gegenüber dem Wert zu Beginn der Behandlung wird auf die Veränderung des Blutvolumens geschlossen. Als Drucksensor kann der venöse Rücklauf- oder arterielle Ansaugdrucksensor dienen. In der Druckschrift wird darauf hingewiesen, dass der Druckabfall an der arteriellen Kanüle eine Funktion des Blutflusses und der Viskosität des Blutes sowie eine Funktion sowohl des Durchmessers als auch der Länge der Kanüle sei. Ferner wird angenommen, dass der Zusammenhang zwischen dem Blutvolumen und der Druckänderung in guter Nährung linear sei.

Der Erfindung liegt die Aufgabe zugrunde, eine Vorrichtung zur extrakorporalen Blutbehandlung mit einer Einrichtung zur Bestimmung des Hämatokrit und/oder Blutvolumens bereitzustellen, die einen verhältnismäßig einfachen Aufbau, aber eine hohe Genauigkeit hat.

Die Lösung dieser Aufgabe erfolgt erfindungsgemäß mit den Merkmalen des Patentanspruchs 1. Vorteilhafte Ausführungsformen sind Gegenstand der Unteransprüche.

Aus sicherheitstechnischen Gründen messen und überwachen die bekannten Dialysegeräte den arteriellen Druck $P_{ar}(t)$ und den venösen Druck $P_{ven}(t)$ im extrakorporalen Blutkreislauf. Im Übrigen wird auch die Rate BPR(t) der Blutpumpe während der Blutbehandlung gemessen bzw. sie ist als Stellwert bekannt. Das erfindungsgemäße Verfahren und die erfindungsgemäße Vorrichtung machen von der ohnehin vorhandenen Druckmessung Gebrauch, so dass der apparative Aufwand verhältnismäßig gering ist.

Dem Grundgedanken der Überwachung des Hämatokrit und Blutvolumens durch die Druckmessung liegt das Folgende zugrunde. Nimmt das relative Blutvolumen während der Blutbehandlung infolge der Ultrafiltration ab, steigt zwangsläufig der Hämatokrit im Blut, da die Dialysemembran für die Blutzellen, nämlich Erythrozyten (7,5 $\mu$m), Leukozyten (1,5 - 20$\mu$m) und Thrombozyten (2,5$\mu$m) nicht durchlässig ist. Weiterhin nimmt die Viskosität mit steigendem Hämatokrit überproportional zu. Da der Strömungswiderstand stark linear mit der Viskosität ansteigt, bedeutet jede durch die Abnahme des Blutvolumens verursachte Steigerung des Hämatokrit eine Mehrbelastung der Blutpumpe, die zum Abfall des arteriellen Drucks (negativ) und der Steigerung des venösen Drucks (positiv) führt, sofern die Blutpumpe mit derselben Rate betrieben wird.

Es hat sich jedoch gezeigt, dass der Zusammenhang zwischen Blutvolumen bzw. Hämatokrit und Druck im extrakorporalen Blutkreislauf nicht nur vom Blutfluss, sondern auch von den Kanülenabmessungen abhängig ist, wobei die Kanüle als die den Druckabfall bestimmende Komponente des extrakorporalen Systems anzusehen ist. Die Erfinder haben erkannt, dass die Länge der Kanüle keinen wesentlichen Einfluss auf den Druck im extrakorporalen Kreislauf hat. Sie haben erkannt, dass allein der Durchmesser der Kanüle bestimmend ist.

Zur Erhöhung der Genauigkeit wird bei der erfindungsgemäßen Vorrichtung der jeweilige Zusammenhang zwischen Hämatokrit bzw. Blutvolumen und Druck für verschiedene Durchmesser der Kanüle und verschiedene Werte des Blutflusses gespeichert. Damit liegen die entsprechenden Daten schon vor der Dialysebehandlung vor. In Abhängigkeit von dem jeweiligen Durchmesser der Kanüle und dem Wert des Blutflusses wird dann der jeweilige Zusammenhang zwischen Hämatokrit bzw. Blutvolumen und Druck ausgewählt und unter Berücksichtigung des ausgewählten Zusammenhangs wird Hämatokrit und/oder Blutvolumen bestimmt. Beispielsweise können die Daten in Form von Kurvenscharen hinterlegt werden, die insbesondere durch diskrete Messwerte beschrieben werden können.

Die erhöhte Genauigkeit ergibt sich daraus, dass nicht nur der Blutfluss während der Behandlung, sondern auch die verwendete Kanüle Berücksichtigung findet.

Wenn von Hämatokrit und Blutvolumen die Rede ist, werden darunter sowohl absolute Werte als auch relative Werte verstanden, die eine relative Veränderung des Blutvolumens zu einem vorgegebenen Anfangswert, beispielsweise der Beginn der Blutbehandlung angeben.

Die Auswertung klinischer Daten hat gezeigt, dass in der Praxis der arterielle Druck, der in der arteriellen Blutleitung stromauf der Blutpumpe gemessen wird, mit dem relativen Blutvolumen viel besser korreliert, als der venöse Druck in der venösen Blutleitung. Dies ist darauf zurückzuführen, dass der venöse Druck sehr viel störanfälliger als der arterielle Druck ist. Bei Dialysemaschinen, die von Bilanzkammern Gebrauch machen, erfasst der venöse Drucksensor die Druckschwankungen, die nicht nur durch die Ultrafiltration, sondern auch durch die Bilanzkammerumschaltung verursacht werden. Auch hat das Luftvolumen bzw. der Pegel in der venösen Tropfkammer eine starke Wirkung auf den Verlauf des venösen Drucksignals. Dem gegenüber ist der arterielle Druck frei von derartigen Druckschwankungen. Zwar wird das arterielle Drucksignal von der Blutpumpenrate beeinflusst, hierbei handelt es sich jedoch um eine eindeutige Störquelle, deren Einfluss auf den arteriellen Druck kompensiert werden kann.

Es hat sich gezeigt, dass der Kanülendurchmesser durch die Auswertung der Druckänderungen im extrakorporalen Blutkreislauf eindeutig bestimmt werden kann. Zur Bestimmung des Kanülendurchmessers wird die aus einer Änderung des Blutflusses resultierende Änderung des Drucks bestimmt und aus der Änderung des Drucks wird auf den Kanülendurchmesser geschlossen. Hierzu werden vorzugsweise bei mindestens jeweils zwei unterschiedlichen Werten des Blutflusses die Drücke gemessen, und die Differenz zwischen den Drücken berechnet. Zur Bestimmung des Kanülendurchmessers wird die Differenz der Drücke mit vorgegebenen für die einzelnen Kanülendurchmesser repräsentativen Wertebereichen verglichen, die abgespeichert werden. Die einzelnen Wertebereiche lassen sich eindeutig den unterschiedlichen Kanülendurchmessern zuordnen. Die Zuordnung zwischen Kanülendurchmesser und Wertebereich kann grundsätzlich durch mehrere Messungen nochmals verifiziert werden.

Weiterhin hat sich gezeigt, dass der Zusammenhang zwischen Hämatokrit bzw. Blutvolumen und Druck für verschiedene Durchmesser der Kanüle und verschiedene Werte des Blutflusses durch eine nicht-lineare Funktion, beispielsweise ein Polynom mit zweiter Ordnung, in ausreichender Näherung beschrieben werden kann. Da der Blutfluss mit der Rate der Blutpumpe korreliert, wird zur Bestimmung des Blutflusses vorzugsweise die Pumpenrate herangezogen, die von der Steuerung der Blutbehandlungsvorrichtung vorgegeben wird.

Wenn der Hämatokrit bestimmt ist, kann das Blutvolumen berechnet werden. Das Blutvolumen berechnet sich zu einem bestimmten Zeitpunkt der Blutbehandlung aus dem Produkt des Hämatokrits zu einem vorausgehenden Zeitpunkt und dem Blutvolumen zu einem vorausgehenden Zeitpunkt geteilt durch den Hämatokrit zu dem bestimmten Zeitpunkt.

Die Einrichtung zur Bestimmung des Hämatokrit und/oder Blutvolumens der erfindungsgemäßen Vorrichtung zur extrakorporalen Blutbehandlung verfügt über eine Speicher- und Auswerteinheit, in der die jeweiligen Zusammenhänge zwischen Hämatokrit bzw. Blutvolumen für die verschiedenen Kanülendurchmesser und Blutflüsse gespeichert sind. Eine derartige Speicher- und Auswerteinheit kann Teil einer Computersteuerung sein, die in den bekannten Blutbehandlungsvorrichtungen ohnehin vorhanden ist. Die Messung des Drucks erfolgt vorzugsweise mit dem ebenfalls ohnehin vorhandenen Drucksensor.

Die Bestimmung des Kanülendurchmessers auf der Grundlage einer Druckmessung ist von eigener erfinderischer Bedeutung. Die Kenntnis des Einflusses der Kanüle kann beispielsweise bei dem aus der DE-C-197 46 377 bekannten Verfahren zur Blutdruckmessung in vorteilhafter Weise dadurch genutzt werden, dass der Einfluss der Blutdichte auf die Pulswellenlaufzeit kompensiert bzw. korrigiert wird, so dass die Blutdruckmessung mit einer höheren Genauigkeit erfolgt.

Im Folgenden wird ein Ausführungsbeispiel einer extrakorporalen Blutbehandlungsvorrichtung mit einer Einrichtung zur Bestimmung des Hämatokrit und/oder Blutvolumens anhand der Figuren näher erläutert.

Es zeigen:

Fig. 1    den Hämatokrit (HKT (%)) als Funktion des arteriellen Drucks ($P_{art}$(mmHg)) für verschiedene Kanülen unterschiedlichen Durchmessers und unterschiedlicher Länge,

Fig. 2    den Hämatokrit (HKT (%)) als Funktion des arteriellen Drucks ($P_{art}$(mmHg)) für verschiedene Kanülen,

Fig. 3    den Hämatokrit (HKT (%)) als Funktion des arteriellen Drucks ($P_{art}$ (mmHg)) für verschiedene Werte des Blutflusses bei einer ersten Kanüle,

Fig. 4    den Hämatokrit (HKT (%)) als Funktion des arteriellen Drucks ($P_{art}$ (mmHg)) für verschiedene Werte des Blutflusses bei einer zweiten Kanüle,

Fig. 5    den Hämatokrit (HKT (%)) als Funktion des arteriellen Drucks ($P_{art}$ (mmHg)) für verschiedene Werte des Blutflusses bei einer dritten Kanüle,

Fig. 6    ein Ausführungsbeispiel einer extrakorporalen Blutbehandlungsvorrichtung mit einer Einrichtung zur Bestimmung des Hämatokrit und/oder Blutvolumens in vereinfachter schematischer Darstellung.

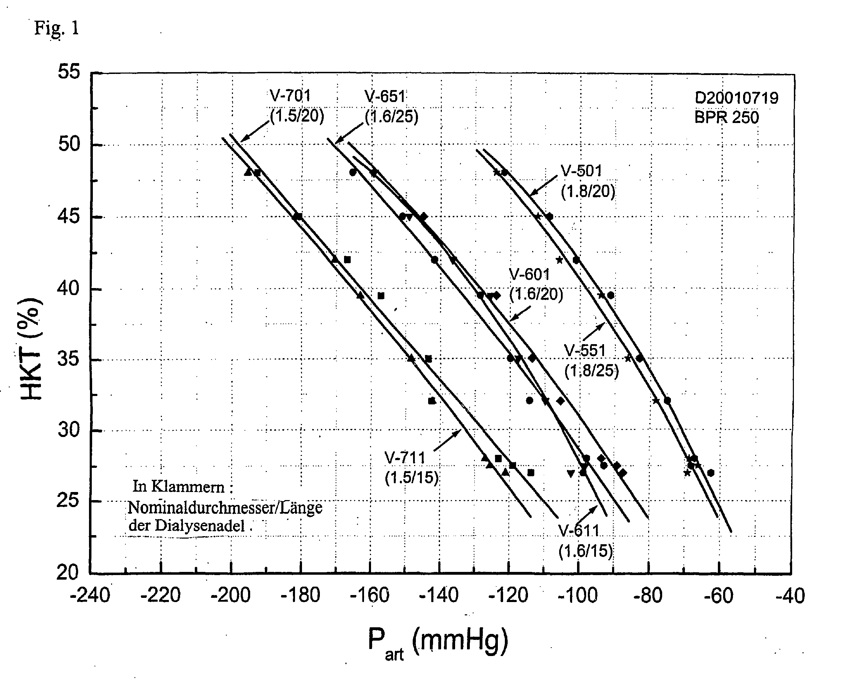

Figur 1 zeigt den Zusammenhang zwischen dem Hämatokrit (HKT (%)) des Blutes und dem Druck in der arteriellen Blutleitung des extrakorporalen Kreislaufs bei einer konstanten Blutpumpenrate BPR von 250 ml/min für sieben verschiedene Dialysekanülen, die sich in Durchmesser und Länge voneinander unterscheiden. Beispielsweise hat die Kanüle mit der Bezeichnung V-711 einen Durchmesser von 1,5 mm und eine Länge von 15 mm. Die anderen Kanülen werden in Figur 1 entsprechend bezeichnet. In Figur 1 ist zu erkennen, dass der Zusammenhang zwischen Hämatokrit und arteriellen Druck nicht linear ist. Er kann aber durch ein Polynom mit zweiter Ordnung in guter Näherung beschrieben werden. Darüber hinaus ist ersichtlich, dass der Zusammenhang zwischen Hämatokrit und Druck stark vom Durchmesser der Kanülen abhängt. Der Einfluss der Länge der Kanülen ist hingegen relativ gering. Daher kann dieser in guter Näherung vernachlässigt werden. Aus diesem Grund ist der Zusammenhang eindeutig nach dem Durchmesser der Kanülen, nämlich 1,5, 1,6 und 1,8 mm gruppiert. Wegen der starken Abhängigkeit des Zusammenhangs vom Durchmesser der Kanüle führt die Messung des Drucks zur Bestimmung des Hämatokrits oder Blutvolumens ohne Kenntnis des Kanülendurchmessers zu ungenauen Ergebnissen.

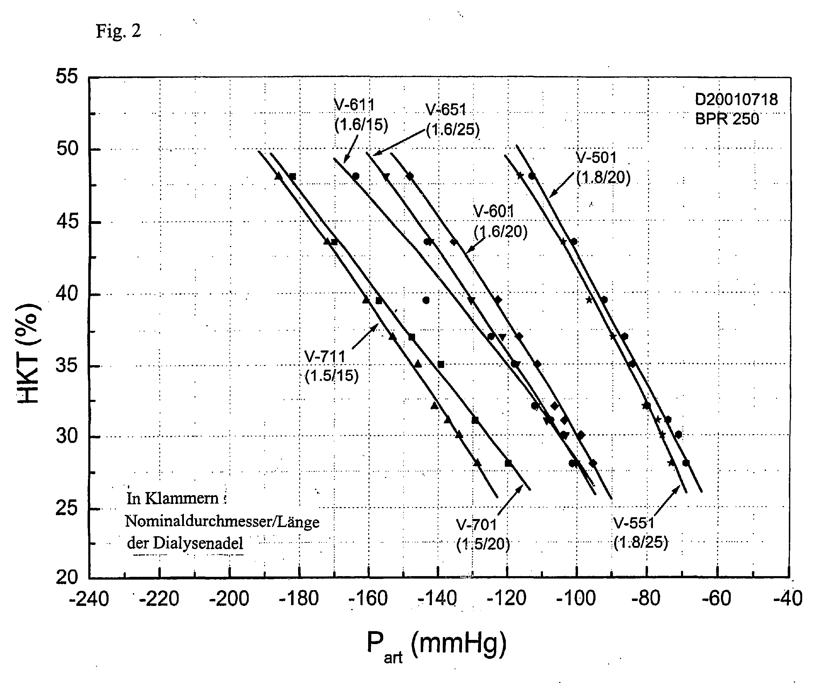

Figur 2 zeigt den Zusammenhang von Hämatokrit und arteriellem Druck einer zweiten Messreihe bei einer Blutflussrate BPR von 250 ml/min. Auch hier ist die Gruppierung nach den Kanülendurchmessern deutlich ausgeprägt.

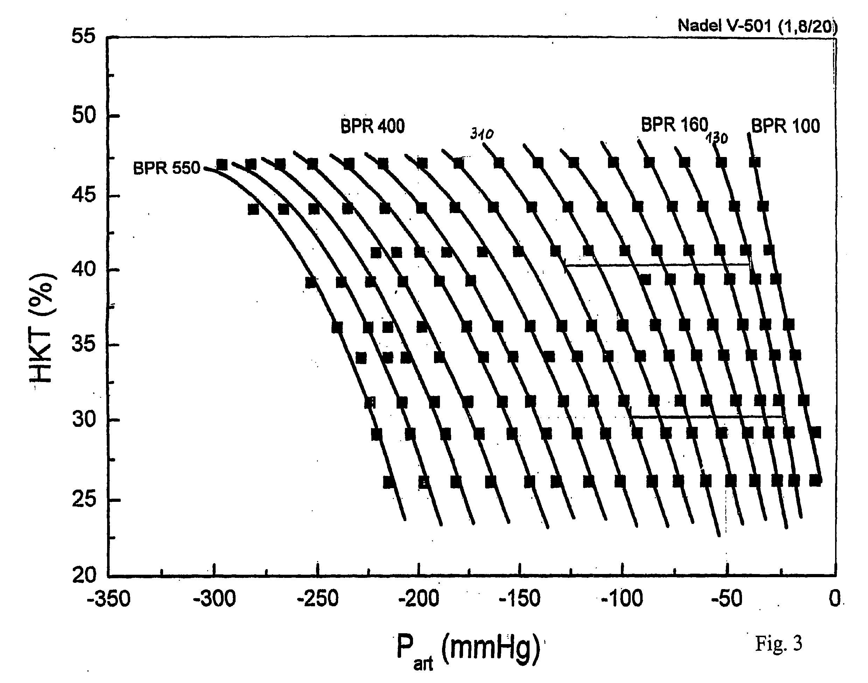

Die Figur 3 zeigt den Zusammenhang zwischen Hämatokrit (HKT (%)) und arteriellem Druck ($P_{art}$(mmHg)) bei einer Nadel mit einem Durchmesser von 1,8 mm und einer Länge von 20 mm für eine Vielzahl von Blutflüssen BPR zwischen 100 ml/min und 550 ml/min. Auch hier ist der Zusammenhang nicht linear. Er kann wiederum durch ein Polynom mit zweiter Ordnung in guter Näherung beschrieben werden. In einem Bereich des Blutflusses von 160 bis 400 ml/min weisen die Kurven für verschiedene Blutflüsse eine ähnliche Steilheit auf. Da sich die Abhängigkeit vom Blutfluss, d. h. der Blutpumpenrate, im Wesentlichen dadurch äußert, dass die Kurven zur x-Achse parallel verschoben werden und die Verschiebung vom Durchmesser der Nadel abhängt, lässt sich der Nadeldurchmesser eindeutig bestimmen. Unter der Annahme, dass der Hämatokrit eines Dialysepatienten im Bereich von 30 % bis 40 % liegt, kann der Durchmesser der Kanüle ohne Kenntnis des Hämatokrit erkannt werden. Die Erkennung erfolgt über Messung der Druckdifferenz bei zwei verschiedenen Blutflüssen, d. h. Blutpumpenraten, wobei typische Werte zwischen 130 ml/min und 310 ml/min liegen.

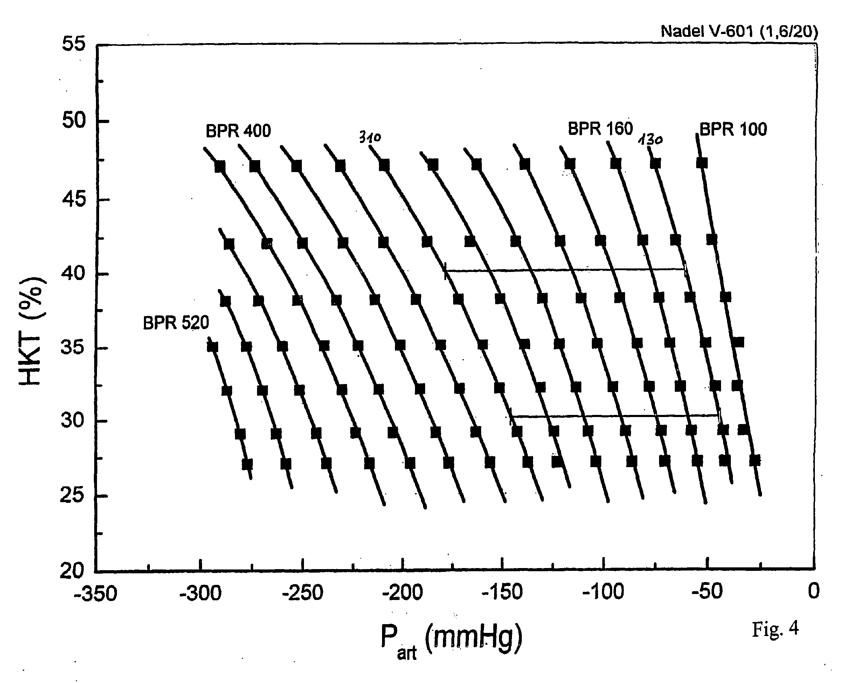

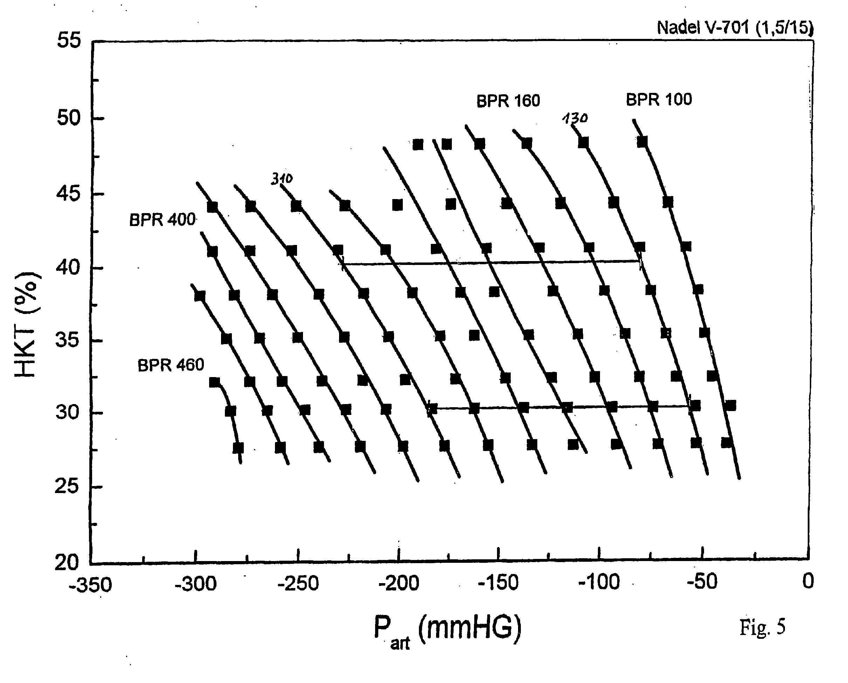

Die Figuren 4 und 5 zeigen die Kurvenscharen einer Nadel mit einem Durchmesser von 1,6 mm und einer Länge von 20 mm bzw. einer Nadel mit einem Durchmesser von 1,5 mm und einer Länge von 15 mm.

Nachfolgend wird die Bestimmung des Kanülendurchmessers anhand der Kurvenscharen in den Figuren 3 - 5 näher erläutert. Hierzu werden bei mindestens zwei vorgegebenen Blutpumpenraten BPR1 und BPR2 die arteriellen Drücke $P_{art1}$ und $P_{art2}$ gemessen. Daraufhin wird die Differenz $\Delta P_{art} = \Delta P_{art1} - P_{art2}$ berechnet, die in den Figuren 3 - 5 als waagerechter Balken dargestellt ist. Für einen HKT-Bereich von circa 30 - 40 % ergeben sich Werte für $\Delta P_{art}$, die den einzelnen Kanülendurchmessern eindeutig zuzuordnen sind. Diese Wertebereiche werden vorher bestimmt und abgespeichert, wobei nach der Messung der Druckänderung eine entsprechende Zuordnung vorgenommen wird.

In der folgenden Tabelle ist die Druckdifferenz $\Delta P_{art}$(mmHg) für die drei Kanülen unterschiedlichen Durchmessers (1,8, 1,6 und 1,5 mm) bei einem Hämatokrit HKT von 30 und 40 % dargestellt. Die Messgrößen lassen sich in die Wertebereiche 70 - 90 mm Hg für einen Kanülendurchmesser von 1,8 mm, 100 bis 120 mm Hg für einen Kanülendurchmesser von 1,6 mm und 130 bis 150 mm Hg für einen Kanülendurchmesser von 1,5 mm gruppieren. Damit kann nach Messung der Druckdifferenz $\Delta P_{art}$ eindeutig entschieden werden, welchen Durchmesser die Kanüle hat. Es zeigt sich, dass der Hämatokrit auf die Eindeutigkeit der Nadeldurchmessererkennung keinen Einfluss hat, wenn er im physiologischen Bereich zwischen 30 und 40 % liegt.

| HKT (%) | $\Delta$ Part (mmHg) | | |
|---|---|---|---|
| | V-501 ($\phi$ 1,8 mm) | V-601 ($\phi$ 1,6 mm) | V-701 ($\phi$ 1,5 mm) |
| 30 | 72 | 102 | 130 |
| 40 | 89 | 118 | 148 |

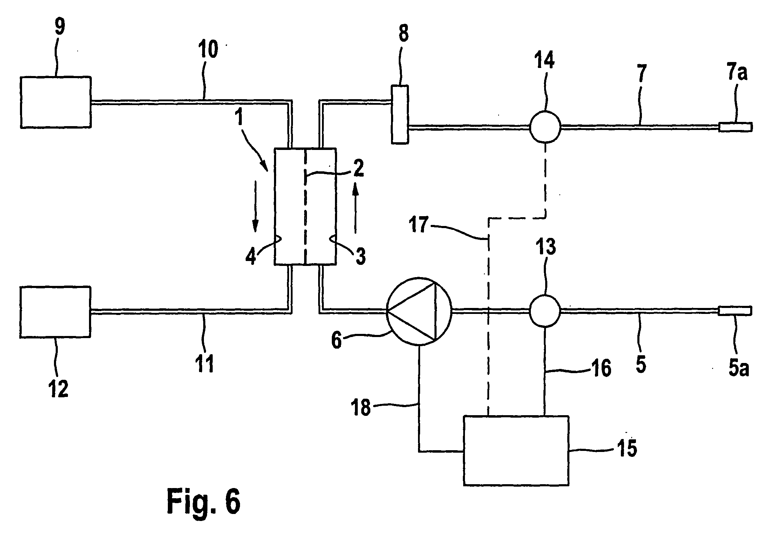

Figur 6 zeigt die wesentlichen Komponenten einer extrakorporalen Blutbehandlungsvorrichtung zusammen mit einer Einrichtung zur Bestimmung des Hämatokrit und/oder Blutvolumens in vereinfachter schematischer Darstellung.

Die Dialysevorrichtung weist als Blutbehandlungseinrichtung einen Dialysator 1 auf, der durch eine semipermeable Membran 2 in eine Blutkammer 3 und eine Dialysierflüssigkeitskammer 4 unterteilt ist. Zu dem Einlass der Blutkammer 3 führt eine arterielle Blutleitung 5, in die eine peristaltische Blutpumpe 6 geschaltet ist. Von der Blutkammer 3 geht eine venöse Blutleitung 7 ab, in die eine Tropfkammer 8 geschaltet ist. An den Enden der arteriellen und venösen Blutleitung 5, 7 sind Kanülen 5a, 7a, angeschlossen, die in den Patienten gestochen werden. Die arterielle und venöse Blutleitung sind Bestandteil eines als Disposable ausgebildeten Schlauchleitungssystems.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang der Dialysierflüssigkeitskammer 4 des Dialysators, während eine Dialysierflüssigkeitsabführleitung 11 von dem Ausgang der Dialysierflüssigkeitskammer zu einem Abfluss 12 führt. Die Dialysevorrichtung verfügt noch über weitere Komponenten, z. B. eine Bilanziereinrichtung und eine Ultrafiltrationseinrichtung etc., die der besseren Übersichtlichkeit halber aber nicht dargestellt sind. Darüber hinaus ist die zentrale Steuereinheit, die Bestandteil der Dialysevorrichtung ist, nicht dargestellt.

Bei der Dialysevorrichtung wird aus sicherheitstechnischen Gründen der arterielle Druck in der arteriellen Blutleitung 5 stromauf der Blutpumpe 6 und der venöse Druck in der venösen Blutleitung stromab der Tropfkammer 8 überwacht. Hierzu ist in der arteriellen Blutleitung 5 ein arterieller Drucksensor 13 und in der venösen Blutleitung 7 ein venöser Drucksensor 14 vorgesehen. Die Einrichtung zur Bestimmung des Hämatokrit und/oder Blutvolumens weist den in der Dialysevorrichtung ohnehin vorhandenen arteriellen Drucksensor 13 sowie eine Speicher- und Auswerteinheit 15 auf. Die Speicher- und Auswerteinheit 15 empfängt über eine Datenleitung 16 das Drucksignal des arteriellen Drucksensors 13. Alternativ kann die Speicher- und Auswerteinheit über eine Datenleitung 17 das Drucksignal des venösen Drucksensors 14 empfangen. Die Datenleitung 17 ist in Figur 6 gestrichelt dargestellt. Darüber hinaus ist die Speicher- und Auswerteinheit 15 über eine Datenleitung 18 mit der Blutpumpe 6 verbunden. Über die Datenleitung 18 wird ein der Blutpumpenrate proportionales Blutpumpensignal übertragen. In der Speicher- und Auswerteinheit sind die in den Figuren 3 - 5 dargestellten Kurvenscharen gespeichert, die den Zusammenhang zwischen Hämatokrit und arteriellem Druck beschreiben. Die Speicher- und Auswerteinheit arbeitet wie folgt.

Während der Dialysebehandlung wird in einer initialen Messung zunächst der Kanülendurchmesser bestimmt, in dem die Blutpumpenrate variiert wird, wobei bei zwei vorgegebene Blutpumpenraten BPR von beispielsweise 310 und 130 mm (Figur 3) die arteriellen Drücke $P_{art1}$ und $P_{art2}$ gemessen werden. Die Speicher- und Auswerteinheit berechnet aus den Messwerten den Betrag der Druckdifferenz $\Delta P_A = P_{art1} - P_{art2}$, der im vorliegenden Beispiel 89 mmHg bei einem Hämatokrit von 40 % beträgt. Grundsätzlich können aber auch Messungen für andere Hämatokritwerte vorgenommen werden, sofern der Hämatokrit im physiologischen Bereich des Patienten und damit zwischen 30 und 40 % liegt. Neben den Kurvenscharen sind in der Speicher- und Auswerteinheit die für den Kanülendurchmesser charakteristischen Wertebereiche von 70 bis 90, 100 bis 120 und 130 bis 150 mmHg gespeichert, die oben beschrieben sind. Die Speicher- und Auswerteinheit nimmt nunmehr eine Zuordnung zwischen der gemessenen Druckdifferenz $\Delta P_{art}$ und den gespeicherten Wertebereichen vor. Da hier die gemessene Druckdifferenz $\Delta P_{art}$ in dem Wertebereich zwischen 70 - 90 mmHg liegt, nimmt die Speicher- und Auswerteinheit an, dass die Kanüle einen Durchmesser von 1,8 mm hat (Figur 3).

Nachdem der Kanülendurchmesser in der initialen Messung festgelegt worden ist, nimmt die Speicher- und Auswerteinheit eine Auswahl zwischen den unterschiedlichen Kurvenscharen vor (Figuren 3 - 5), die jeweils den Zusammenhang von Hämatokrit und arteriellem Druck für den jeweiligen Nadeldurchmesser beschreiben. Hier wählt die Speicher- und Auswerteinheit die Kurvenscharen gemäß Figur 3 aus, die für den vorliegenden Nadeldurchmesser von 1,8 mm repräsentativ sind.

Nach der Auswahl der passenden Kurvenschar bestimmt die Speicher- und Auswerteinheit aus der passenden Kurvenschar den passenden Hämatokrit in Abhängigkeit von der Blutpumpenrate BPR(t) unter Berücksichtigung des Durchmessers der verwendeten Kanüle mit hoher Genauigkeit, ohne dass der Durchmesser der verwendeten Kanüle manuell eingegeben werden bräuchte. Wird beispielsweise mit dem arteriellen Drucksensor ein arterieller Druck von -100 mmHg gemessen, so ergibt sich bei einer Blutpumpenrate von 310 mm ein Hämatokrit von etwa 33 % (Fig. 3). Mit abnehmender Blutpumpenrate nimmt der Hämatokrit entsprechend der Kurvenschar zu.

Die Bestimmung des Blutvolumens erfolgt, nachdem der Hämatokrit ermittelt worden ist. Das Blutvolumen zu einem bestimmten Zeitpunkt der Blutbehandlung RBV(t) berechnet sich aus dem Hämatokrit HKT nach der folgenden Gleichung:

$$RBV(t) = \frac{HKT\,(t_0)\;RBV\,(t_0)}{HKT\,(t)}$$

wobei RBV(t) das Blutvolumen zum Zeitpunkt t,
HKT(t) der Hämatokrit zum Zeitpunkt t und RBV $(t_0)$ bzw. HKT $(t_0)$ das Blutvolumen und der Hämatokrit zu einem beliebigen Zeitpunkt $t_0$ sind, der vor dem Zeitpunkt t liegt.

Da zu Beginn der Dialysebehandlung RBV$(t_0)$ = 1 ist, kann die Speicher- und Auswerteinheit RBV(t) relativ zu diesem Zeitpunkt bestimmen. Andererseits kann die obige Gleichung auch für zwei beliebige Zeitpunkte $t_0$ und t verwendet werden, wenn $t_0$ nicht mit dem Behandlungsbeginn zusammenfällt und damit $RBV(t_0)$ nicht notwendigerweise 1 ist. Sollte $RBV(t_0)$ nicht bekannt sein, kann die Speicher- und Auswerteinheit wiederum relative Änderungen von RBV nach der obigen Gleichung im Vergleich zu einem Wert von RBT $(t_0)$ von 1 bestimmen.

**Patentansprüche**

1. Vorrichtung zur extrakorporalen Blutbehandlung mit einem extrakorporalen Blutkreislauf, der eine Blutpumpe (6) und eine arterielle Kanüle (5a) und arterielle Schlauchleitung (5) zum Entnehmen von Blut und eine venöse Kanüle (7a) und venöse Schlauchleitung (7) zum Rückführen von Blut aufweist, mit einer Einrichtung zum Bestimmen des Hämatokrit und/oder Blutvolumens, die einen Drucksensor (13, 14) zum Messen des Drucks im extrakorporalen Kreislauf und eine Speicher- und Auswerteinheit (15) aufweist, die derart ausgebildet ist, dass bei einer Änderung des Drucks auf eine Änderung des Hämatokrit bzw. Blutvolumens geschlossen wird, **dadurch gekennzeichnet, dass** in der Speicher- und Auswerteinheit (15) der jeweilige Zusammenhang zwischen Hämatokrit HKT bzw. Blutvolumen RBV und Druck P im extrakorporalen Kreislauf für verschiedene Kanülendurchmesser und verschiedene Blutflusswerte gespeichert ist und dass die Speicher- und Auswerteinheit (15) derart ausgebildet ist, dass für den jeweiligen Kanülendurchmesser und Blutfluß der jeweilige Zusammenhang ausgewählt und unter Berücksichtigung des ausgewählten Zusammenhangs Hämatokrit und/oder Blutvolumen bestimmt wird.

2. Vorrichtung nach Anspruch 1, **dadurch gekennzeichnet, dass** der Drucksensor (13) in der arteriellen Blutleitung (5) stromauf der Blutpumpe (6) angeordnet ist.

3. Vorrichtung nach Anspruch 1 oder 2, **dadurch gekennzeichnet, dass** die Speicher- und Auswerteinheit (15) derart ausgebildet ist, dass zur Bestimmung des Kanülendurchmesser, die aus einer Änderung des Blutflusses resultierende Änderung des arteriellen Drucks bestimmt und aus der Änderung des arteriellen Drucks auf den Kanülendurchmesser geschlossen wird.

4. Vorrichtung nach Anspruch 3, **dadurch gekennzeichnet, dass** die Speicher- und Auswerteinheit (15) derart ausgebildet ist, dass bei mindestens jeweils zwei unterschiedlichen Werten des Blutflusses die Drücke $P_{art1}$ und $P_{art2}$ gemessen und die Differenz $\Delta P_{art} = P_{art1} - P_{art2}$ von den Drücken $P_{art1}$ und $P_{art2}$ berechnet wird, wobei zur Bestimmung des Kanülendurchmessers die Differenz $\Delta P_{art}$ mit vorgegebenen für die einzelnen Kanülendurchmesser repräsentativen Wertebereichen verglichen wird.

5. Vorrichtung nach einem der Ansprüche 1 bis 4, **dadurch gekennzeichnet, dass** die Speicher- und Auswerteinheit (15) derart ausgebildet ist, dass der Zusammenhang zwischen Hämatokrit HKT bzw. Blutvolumen RBV und Druck für verschiedene Kanülendurchmesser und verschiedene Blutflusswerte durch eine nicht-lineare Funktion beschrieben wird.

6. Vorrichtung nach einem der Ansprüche 1 bis 5, **dadurch gekennzeichnet, dass** die Speicher- und Auswerteinheit (15) derart ausgebildet ist, dass zur Bestimmung des Blutflusses die Pumpenrate BPR der Blutpumpe (6) bestimmt wird.

7. Vorrichtung nach einem der Ansprüche 1 bis 6, **dadurch gekennzeichnet, dass** die Speicher- und Auswerteinheit (15) derart ausgebildet ist, dass das Blutvolumen RBV aus dem Hämatokrit HKT bestimmt wird.

8. Vorrichtung nach Anspruch 7, **dadurch gekennzeichnet, dass** die Speicher- und Auswerteinheit (15) derart ausgebildet ist, dass das Blutvolumen RBV zu einem bestimmten Zeitpunkt t der Blutbehandlung aus dem Produkt HKT $(t_0)$ RBV $(t_0)$ des Hämatokrit $HKT(t_0)$ zu einem vorausgehenden Zeitpunkt $t_0$ und des Blutvolumens $RBV(t_0)$ zu einem vorausgehenden Zeitpunkt $t_0$, geteilt durch den Hämatokrit $HKT(t)$ zu dem bestimmten Zeitpunkt t berechnet wird.

**Claims**

1. An apparatus for extracorporeal blood treatment with an extracorporeal blood circuit, which has a blood pump (6) and an arterial cannula (5a) and an arterial flexible-tube line (5) for taking blood and a venous cannula (7a) and venous flexible-tube line (7) for feeding back blood, with a device for determining the hematocrit and/or blood volume, which has a pressure sensor (13, 14) for measuring the pressure in the extracorporeal circuit and a memory and evaluation unit (15) which is designed in such a way that a change in the hematocrit or blood volume is deduced from a change in the pressure, **characterised in that** the respective relationship between hematocrit HKT or blood volume RBV and pressure P in the extracorporeal circuit for various cannula diameters and various blood-flow values is stored in the memory and evaluation unit (15) and that the memory and evaluation unit (15) is designed in such a way that the respective relationship is selected for the respective cannula diameter and the hematocrit and/or blood volume is determined taking account of the selected relationship.

2. The apparatus according to claim 1, **characterised in that** the pressure sensor (13) is disposed in the arterial blood line (5) upstream of the blood pump (6).

3. The apparatus according to claim 1 or 2 **characterised in that** the memory and evaluation unit (15) is designed in such a way that, in order to determine the cannula diameter, the change in the arterial pressure resulting from a change in the blood flow is determined and the cannula diameter is deduced from the change in the arterial pressure.

4. The apparatus according to claim 3, **characterised in that** the memory and evaluation unit (15) is designed in such a way that the pressures $P_{art1}$ and $P_{art2}$ are measured at at least two different values of the blood flow in each case and the difference $\Delta P_{art} = P_{art1} - P_{art2}$ is calculated from the pressures $P_{art1}$ and $P_{art2}$, whereby the difference $\Delta P_{art}$ is compared with predetermined value ranges representative of the individual cannula diameters in order to determine the cannula diameter.

5. The apparatus according to any one of claims 1 to 4, **characterised in that** the memory and evaluation unit (15) is designed in such a way that the relationship between hematocrit HKT or blood volume RBV and pressure for various cannula diameters and various blood-flow values is described by a non-linear function.

6. The apparatus according to any one of claims 1 to 5, **characterised in that** the memory and evaluation unit (15) is designed in such a way that the pumping rate BPR of the blood pump (6) is determined in order to determine the blood flow.

7. The apparatus according to any one of claims 1 to 6, **characterised in that** the memory and evaluation unit (15) is designed in such a way that the blood volume RBV is determined from the hematocrit HKT.

8. The apparatus according to claim 7, **characterised in that** the memory and evaluation unit (15) is designed in such a way that the blood volume RBV is calculated at a specified time t of the blood treatment from the product HKT $(t_0)$ RBV $(t_0)$ of the hematocrit HKT $(t_0)$ at a preceding time $t_0$ and the blood volume RBV $(t_0)$ at a preceding time $t_0$, divided by the hematocrit HKT(t) at the specified time t.

## Revendications

1. Dispositif de traitement sanguin extra-corporel avec une circulation sanguine extracorporelle qui présente une pompe de sang (6) et une canule artérielle (5a) et une conduite flexible artérielle (5) pour évacuer le sang et une canule veineuse (7a) et une conduite flexible veineuse (7) pour réacheminer le sang, avec un dispositif pour déterminer le taux d'hématocrite et/ou du volume sanguin qui présente un capteur de pression (13, 14) pour mesurer la pression dans la circulation extracorporelle et une unité de stockage et d'évaluation (15) qui est constituée de telle sorte qu'en cas de modification de la pression, une modification du taux d'hématocrite ou du volume sanguin est indiquée, **caractérisé en ce que** dans l'unité de stockage et d'évaluation (15) le rapport respectif entre le taux d'hématocrite HKT ou le volume sanguin RBV et la pression P dans la circulation extracorporelle est enregistré pour divers diamètres de canule et diverses valeurs de flux sanguin et que l'unité de stockage et d'évaluation (15) est conçue de telle sorte que pour le diamètre de canule et le flux sanguin respectifs, le rapport respectif soit choisi et soit déterminé en tenant compte du rapport choisi de l'hématocrite et/ou du volume sanguin.

2. Dispositif selon la revendication 1, **caractérisé en ce que** le capteur de pression (13) dans la conduite de sang artérielle (5) est disposé en amont de la pompe de sang (6).

3. Dispositif selon la revendication 1 ou 2, **caractérisé en ce que** l'unité de stockage et d'évaluation (15) est conçue de telle sorte que, pour déterminer le diamètre de canules qui détermine une modification de la pression artérielle résultant d'une modification du flux sanguin et de la modification de la pression artérielle, le diamètre de canules est indiqué.

4. Dispositif selon la revendication 3, **caractérisé en ce que** l'unité de stockage et d'évaluation (15) est conçue de telle sorte qu'avec au moins deux valeurs respectivement différentes du flux sanguin, les pressions $P_{art1}$ et $P_{art2}$ sont mesurées et la différence $\Delta P_{art} = P_{art1} - P_{art2}$ est calculée à partir des pressions $P_{art1}$ et $P_{art2}$, sachant que pour déterminer le diamètre de canule, la différence $\Delta P_{art}$ est comparée avec les valeurs représentatives préétablies pour le diamètre de canule individuel.

5. Dispositif selon l'une quelconque des revendications 1 à 4, **caractérisé en ce que** l'unité de stockage et d'évaluation (15) est conçue de telle sorte que le rapport entre hématocrite HKT ou volume sanguin RBV et la pression est décrit, pour différents diamètres de canule et différentes valeurs de flux sanguin, par une fonction non linéaire.

6. Dispositif selon l'une quelconque des revendications 1 à 5, **caractérisé en ce que** l'unité de stockage et d'évaluation (15) est conçue de telle sorte que pour déterminer le flux sanguin, le taux de pompage BPR de la pompe de sang (6) est déterminé.

7. Dispositif selon l'une quelconque des revendications 1 à 6, **caractérisé en ce que** l'unité de stockage et d'évaluation (15) est conçue de telle sorte que le volume sanguin RBV est déterminé à partir du taux d'hématocrite HKT.

8. Dispositif selon la revendication 7, **caractérisé en ce que** l'unité de stockage et d'évaluation (15) est conçue de telle sorte que le volume sanguin RBV à un temps t donné du traitement sanguin est calculé à partir du produit HKT $(t_0)$ RBV $(t_0)$ du taux d'hématocrite HKT $(t_0)$ à un temps $t_0$ précédent et du volume sanguin RBV $(t_0)$ à un temps $t_0$ précédent, divisé par le taux d'hématocrite HKT (t) au temps t déterminé.

Fig. 1

Fig. 2

EP 1 480 695 B1

Fig. 3

Fig. 4

EP 1 480 695 B1

Fig. 5

Fig. 6

EP 1 480 695 B1

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19746377 C

- DE 4024434 A